Europäisches Patentamt

European Patent Office

Office européen des brevets

(19)

(11) **EP 1 449 919 A1**

(12) **EUROPEAN PATENT APPLICATION**
published in accordance with Art. 158(3) EPC

(43) Date of publication:
**25.08.2004 Bulletin 2004/35**

(51) Int Cl.[7]: **C12N 15/09**, C12N 5/10,
C12P 21/02

(21) Application number: **02788693.6**

(22) Date of filing: **29.11.2002**

(86) International application number:
**PCT/JP2002/012522**

(87) International publication number:
**WO 2003/046174 (05.06.2003 Gazette 2003/23)**

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(30) Priority: **29.11.2001 JP 2001363734**

(71) Applicant: **KYOWA HAKKO KOGYO CO., LTD.
Chiyoda-ku, Tokyo 100-8185 (JP)**

(72) Inventors:
• **Konno, Yoshinobu Tokyo Res. Lab.
Machida-shi, Tokyo 194-8533 (JP)**

• **Aski, Motoi Head Office
Kyowa Hakko Kogyo Co., Ltd
Tokyo 100-8185 (JP)**
• **Takasugi, Hiroshi
Technical Research Laboratories
Hofu-shi, Yamaguchi 747-8522 (JP)**
• **Takagishi, Masakazu
Tokyo Research Laboratories
Machida-shi, Tokyo 194-8533 (JP)**

(74) Representative: **Harding, Charles Thomas
D. Young & Co.
21 New Fetter Lane
London EC4A 1DA (GB)**

(54) **PROCESS FOR PRODUCING SUBSTANCE**

(57)    The present invention relates to a process for producing a substance, which comprises culturing an animal cell having an ability to produce the substance in a medium containing a ubiquinone such as Q10 until the substance is produced and accumulated in the culture, and recovering the substance therefrom, and a method of improving the productivity of a substance, which comprises culturing an animal cell having an ability to produce the substance in a medium containing a ubiquinone.

**Description**

Technical Field

The present invention relates to a process for producing substances by culturing animal cells and a method of improving the productivity of substances.

Background Art

The process for producing substances by culturing animal cells or recombinant animal cells has been carried out widely. The process is frequently applied to the production of pharmaceutical products, in particular, and thus the demand for the improved process thereof will increase hereafter.

Because many of substances useful as pharmaceutical products generally have higher order structures or sugar chains, the animal cell culture by which physiologically active substances can be acquired is employed to produce such substances.

However, the animal cell culture is problematic in that the growth rates of animal cells are slow; the media for use in the culture are expensive; the productivity per medium is low; and the productivity per cell is low. Therefore, various studies have been made so as to overcome the problems. As the method of improving the substance productivity, for example, methods by changing temperature (Japanese Published Unexamined Patent Application No. 75077/97) and by adding n-butyric acids (Japanese Published Unexamined Patent Application No. 9968/96), iron citrate or ethanolamine (Japanese Published Unexamined Patent Application No. 91786/92) have been known.

For animal cell culture, the following media have been used; a medium prepared by adding animal serum such as calf fetus serum to a basal medium comprising amino acids, vitamins, sugars, salts and the like; or a serum-free medium prepared by adding insulin, transferrin, 2-ethanolamine, sodium selenite, bovine serum albumin, cell growth factors and the like as alternatives of animal serum to the basal medium. Because sera, insulin, cell growth factors and the like are expensive, the resulting media are costly, leading to the elevation of the production cost of useful substances.

When desired substances produced by culturing animal cells, such as an antibody, are used for diagnostic agents administrated into human bodies or therapeutic agents, the concentration of protein impurities and the like contaminated therein should be reduced to no safety concerns. Hence, it is required to separate and remove the components added to media, such as sera, insulin, transferrin and cell growth factors from the desired substances produced.

Still further, it has been demanded in recent years that the use of animal-derived raw materials typically including animal sera and the alternatives of animal sera as media components should be avoided as much as possible. However, no addition of such materials frequently causes prominent reduction of the productivity of useful substance in many of animal cells.

Since ubiquinone has a significant role in the mitochondrial respiratory chain during cell growth as well as an anti-oxidative effect, ubiquinone is used in health drinks or pharmaceutical products. Additionally, it has been known that ubiquinone added to media promotes the growth of HeLa cells and murine fibroblast cells [Protoplasma, 184, 214 (1995)] and the growth of bovine embryo cells [Biol. Reprod., 61, 541 (1999)].

However, no relationship between ubiquinone and the productivity of a substance has been known.

Disclosure of the Invention

It is an object of the present invention to provide a process for producing a substance efficiently by use of an animal cell.

The present invention relates to the following (1) to (18).

(1) A process for producing a substance, which comprises culturing an animal cell having an ability to produce the substance in a medium containing a ubiquinone thereby to produce and accumulate the substance in the culture, and recovering the substance therefrom.

(2) The process according to (1), wherein the ubiquinone is Q10.

(3) The process according to (1) or (2), wherein the animal cell is a cell of an animal belonging to mammals.

(4) The process according to any one of (1) to (3), wherein the animal cell is a myeloma cell, a cell derived from the myeloma cell or an ovarian cell.

(5) The process according to any one of (1) to (4), wherein the cell is a cell transformed with a recombinant vector carrying a gene involved in the production of the substance.

(6) The process according to any one of (1) to (5), wherein the medium is a serum-free medium or a protein-free medium.

(7) The process according to any one of (1) to (6), wherein the culturing is carried out by batch culture, repeated batch culture, fed-batch culture or perfusion culture.

(8) The process according to any one of (1) to (7), wherein the substance is selected from an immunologically functional molecule, a biocatalyst molecule, a structure-forming molecule, a structure-maintaining molecule or a vaccine.

(9) The process according to (8), wherein the immunologically functional molecule is an antibody.

(10) A method of improving the productivity of a substance, which comprises culturing an animal cell having an ability to produce the substance in a medium containing a ubiquinone.

(11) The method according to (10), wherein the ubiquinone is Q10.

(12) The method according to (10) or (11), wherein the animal cell is a cell of an animal belonging to mammals.

(13) The method according to any one of (10) to (12), wherein the animal cell is a myeloma cell, a cell derived from the myeloma cell or an ovarian cell.

(14) The method according to any one of (10) to (13), wherein the cell is a cell transformed with a recombinant vector carrying a gene involved in the production of the substance.

(15) The method according to any one of (10) to (14), wherein the medium is a serum-free medium or a protein-free medium.

(16) The method according to any one of (10) to (15), wherein the culturing is carried out by batch culture, repeated batch culture, fed-batch culture or perfusion culture.

(17) The method according to any one of (10) to (16), wherein the substance is selected from an immunologically functional molecule, a biocatalyst molecule, a structure-forming molecule, a structure-maintaining molecule or a vaccine.

(18) The method according to (17), wherein the immunologically functional molecule is an antibody.

Any medium used in the animal cell culture can be used as the medium of the present invention, except for necessity of adding a ubiquinone at preferably 1 to 10,000 μM, more preferably 10 to 1,000 μM, still more preferably 100 to 1,000 μM to the medium.

As the ubiquinone (referred to as Coenzyme Q, namely 2,3-dimethoxy-5-methyl-6-polyprenyl-1,4-benzoquinone), preferably, Qn ("n" represents the number of isoprene units) with "n" of 1 to 12 is used; more preferably, Q6, Q8 and Q10 are used; and still more preferably, Q10 is used.

The term ubiquinone contains an analogous substance of ubiquinone artificially synthesized so as to enhance the water solubility, such as polyethylene glycol 5000-modified Q10 (WO 96/17626).

Ubiquinone may be added to the medium as it is. Because of the high lipid solubility, it is preferable to be added after improving the water solubility. The method of improving the water solubility of ubiquinone includes a method by use of cyclodextrin [Acta Pol. Pharm., 53, 193 (1996)] or by homogenizing under heating [Biol. Reprod., 61, 541 (1999)].

Commercially available products containing ubiquinone include SANOMIT (trade name; manufactured by MSE Pharmazeutica GmbH.)

The amount of ubiquinone to be added to the medium may appropriately be selected, depending on the type of the animal cell, the type of a desired substance, the timing of adding ubiquinone and the like. The concentration of ubiquinone in the medium is preferably 1 to 10,000 μmol/L, more preferably 10 to 1,000 μmol/L and still more preferably 100 to 1,000 μmol/L.

As the medium for use in animal cell culture, any of serum-containing media, serum-free media and protein-free media may be used. Preferably, serum-free media or protein-free media with no content of contaminants derived from animal proteins are used.

As the serum-containing medium, a basal medium used in general animal cell culture and supplemented with serum can be used.

The basal media include RPMI 1640 medium [J. Am. Med. Assoc., 199, 519 (1967)], Eagle's MEM [Science, 122, 501 (1952)], Dulbecco's modified Eagle's medium (DMEM) [Virology, 8, 396 (1959)], 199 medium [Proc. Soc. Exp. Biol. Med., 73, 1 (1950)], F12 medium [Proc. Natl. Acad. Sci. USA., 53, 288 (1965)], Iscove's modified Dulbecco's Medium (IMDM) [J. Exp. Med., 147, 923 (1978)] and mixed media of these media. Preferably, RPMI 1640 medium, DMEM, F12 medium, IMDM and the like can be used.

As the serum-containing medium, a basal medium supplemented with serum from cow, horse or the like can be used at an appropriate amount, usually around 5 to 10 %.

As the serum-free medium, a basal medium used for the serum medium and supplemented with physiologically active substances, nutrient factors, and carbon sources, nitrogen sources and the like which are assimilable by animal cells, instead of serum is used.

Nutrient factors, physiologically active substances or the like required for animal cell growth, if necessary, are added to the serum-free medium. Preferably, these additives are preliminarily contained in the medium prior to culturing.

The nutrient factors include glucose, amino acids and vitamins.

The amino acids include L-alanine, L-arginine, L-asparagine, L-aspartic acid, L-cystine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, andL-valine, which are used singly or in combination of two or more thereof.

The vitamins include d-biotin, D-pantothenic acid, choline, folic acid, myo-inositol, niacin amide, pyridoxal, riboflavin, thiamine, cyanocobalamine, and DL-α-tocopherol, which are used singly or in combination of two or more thereof.

The physiologically active substances include insulin, transferrin, serum albumin and a growth factor-containing serum fraction.

For a medium free of animal-derived materials, examples of a substance to be added instead of an animal-derived material such as serum albumin or a serum fraction include a physiologically active protein substance produced by genetic engineering techniques, hydrolyzed soybean and cholesterol lipid free of animal-derived materials.

The protein-free media include ADPF medium (Animal-derived-protein-free medium; manufactured by Hy-Clone Laboratories Inc.), CD-Hybridoma medium (manufactured by Invitrogen Corporation), and CD-CHO medium (manufactured by Invitrogen Corporation).

In case of the long-term or high-density culture, media containing high concentrations of amino acids and vitamins, for example, a medium constituted by RPMI 1640 medium, DMEM and F12 medium at a ratio of 1:1:1, a medium constituted by DMEM and F12 medium at a ratio of 1:1, Hybridoma-SFM (manufactured by Invitrogen Corporation) and the like are preferably used.

As the animal cells in the present invention, cells of animals belonging to any of mammals, birds, reptiles, amphibians, fishes, and insects may be used so long as they have an ability to produce substances. Preferably, cells of animals belonging to mammals are preferably used. Suitable cells of animals belonging to mammals include myeloma cells, cells derived from the myeloma cells, ovarian cells, kidney cells, blood cells, uterine cells, connective tissue cells and mammary gland cells. Especially, myeloma cells, cells derived from the myeloma cells or ovarian cells are preferable. When the desired substance is an antibody, the animal cell is preferably an antibody-producing cell.

Specific examples of the cells of animals belonging to mammals include HL-60 (ATCC No.: CCL-240), HT-1080 (ATCC No.: CCL-121), HeLa (ATCC No.: CCL-2), 293 (ECACC No.: 85120602), Namalwa (ATCC No.: CRL-1432) and Namalwa KJM-1 (Cytotechnology, 1, 151 (1988)] which are human cell lines; Vero (ATCC No.: CCL-81) and COS-7 (ATCC No.: CRL-1651) which are monkey cell lines; C127I (ATCC No.: CRL-1616), Sp2/0-Ag14 (ATCC No.: CRL-1581) and NIH/3T3 (ATCC No.: CRL-15) which are mouse cell lines; NSO (ECACC No.: 85110503), Y3-Ag1.2.3. (ATCC No.: CRL-1631), YO (ECACC No.: 85110501) and YB2/0 (ATCC No.: CRL-1662 ) which are rat cell lines; CHO (ECACC No.: 85050302), CHO/dhfr- (ATCC No. CRL-9096), CHO/DG44 [Proc. Natl. Acad. Sci USA, 77, 4216 (1980)] and BHK21 (clone 13) (ECACC No.: 85011433) which are hamster cell lines; and MDCK (ATCC No.: CCL-34) which is a dog cell line. The cells of animals belonging to birds, the cells of animals belonging to fishes, and the cells of animals belonging to insects include chicken cell line SL-29 (ATCC No. : CRL-1590), zebra fish cell line ZF4 (ATCC No.: CRL-2050) and moth (Spodoptera frugiperda) cell line Sf9 (ATCC No.: CRL-1711), respectively. Additionally, the primary culture cells for use in vaccine production include primary monkey kidney cells, primary rabbit kidney cells, primary chicken embryo cells and primary quail embryo cells.

Examples of the myeloma cells or the cells derived from the myeloma cells include rat myeloma cell lines such as NSO, Y3-Ag1.2.3. and YO, and hybridoma cells of myeloma and other cells such as YB2/0 which is a hybridoma cell line of rat myeloma cell line Y3-Ag1.2.3. and a rat spleen cell, and Sp2/0-Ag14 which is a hybridoma cell line of mouse myeloma cell line P3X63Ag8 and a mouse spleen cell. Examples of the ovarian cells include CHO, CHO/dhfr- and CHO/DG44 which are Chinese hamster ovarian cell lines. Examples of the kidney cells include 293 which is a human kidney cell line, Vero and COS-7 which are monkey kidney cell lines, BHK 21 (clone 13) which is a hamster kidney cell line, and MDCK which is a dog kidney cell line. Examples of the blood cells include HL-60 which is a human promyelocytic cell line, and Namalwa and Namalwa KJM-1 which are human B cell lines. Examples of the uterine cells include Hela which is a human uterine cervix cell line. Examples of the connective tissue cells include HT-1080 which is a human fibroblast cell line, and NIH/3T3 which is a mouse fibroblast cell line. Examples of the mammary gland cells include C127I which is a mouse mammary gland cell line.

As the animal cells of the present invention, animal cells producing a desired substance, cells treated with a mutation process so as to acquire the ability of producing a desired substance, cells trans formed with a recombinant vector carrying the gene involved in the production of a desired substance, and hybridomas between a B cell obtained from a non-human mammal immunized with an antigen and a myeloma cell are preferably used.

The cell transformed with a recombinant vector carrying the gene involved in the production of a desired substance can be prepared by transfecting a recombinant vector containing the DNA involved in the production of the substance and a promoter into a host cell. As the host cell, the foregoing animal cells are used.

As the DNA involved in the production of a desired substance, DNAs encoding the desired substance such as protein, DNAs encoding an enzyme capable of catalyzing the biosynthesis of the substance can be used.

Examples of the vectors for use in preparing the recombinant vector include pcDNA 3.1(+) (manufactured by Invitrogen Corporation), pAGE107 [Japanese Published Unexamined Patent Application No. 22979/91, Cytotechnology, 3, 133 (1990)], pAS3-3 (Japanese Published Unexamined Patent Application No. 227075/90), pCDM8 [Nature, 329, 840 (1987)], pcDNA6/HisA (manufactured by Invitrogen Corporation), pREP4 (manufactured by Invitrogen Corporation), pAGE103 [J. Biochem. , 101, 1307 (1987)] and pAGE210 (WO 97/10354).

As the promoter, any of promoters capable of functioning in the animal cells for use in the present invention can be used. The examples of the promoters include the IE (immediate early) gene promoter of cytomegalovirus (CMV), the early promoter of SV40, retrovirus promoter, metallothionein promoter, heat shock promoter and SRα promoter. An enhancer of the human CMV IE gene may also be used in combination with the promoter.

As the method for transfecting a recombinant vector into a host cell, any of methods for transfecting DNAs into host cells can be used. The examples of the methods include the electroporation method [Cytotechnology, 3,133 (1990)], the calcium phosphate method (Japanese Published Unexamined Patent Application No. 227075/90) and the lipofection method [Proc. Natl. Acad. Sci. USA, 84, 7413 (1987), Virology, 52, 456 (1973)].

The transformed cell includes transformed cells KM-871 (FERM BP-3512, Japanese Published Unexamined Patent Application No. 304989/93), 7-9-51 (FERM BP-6691, WO 01/29246) and 61-33γ (FERM BP-7325, WO 01/29246) producing a chimeric humanized anti-GD3 antibody; transformed cell KM 8871 (FERM BP-6790, WO 01/23432) producing a CDR-grafted humanized anti-GD3 antibody; transformed cells KM2760 (FERM BP-7054, WO 01/65754), Nega-13 (FERM BP-7756, WO 02/31140), 503LCA500D (FERM BP-8239) and NSO/LCA-CCR4 (FERM BP-7964) producing a chimeric humanized anti-CCR4 antibody; transformed cells KM1399 (FERM BP-5650, WO 97/10354) and KM7399 (FERM BP-5649, WO 97/10354) producing a chimeric humanized anti-IL-5 receptor α-chain antibody; transformed cells KM8399 (FERM BP-5648, WO 97/10354) and KM9399 (FERM BP-5647, WO 97/10354) producing a CDR-grafted humanized anti-IL-5 receptor α-chain antibody; and transformed cells KM8966 (FERM BP-5105, Japanese Patent Published Unexamined Application No. 257893/98), KM8967 (FERM BP-5106, Japanese Patent Published Unexamined Application No. 257893/1998), KM8969 (FERM BP-5527, Japanese Patent Published Unexamined Application No. 257893/1998) and KM8970 (FERM BP-5528, Japanese Patent Published Unexamined Application No. 257893/1998) producing a CDR-grafted humanized anti-GM2 antibody.

As the culture process for animal cell, any culture process efficiently producing a desired substance can be used, including batch culture, repeated batch culture, fed-batch culture and perfusion culture. Preferably, fed-batch culture or perfusion culture is used.

1. Batch culture

Batch culture is usually carried out in a medium for use in animal cell culture at pH 6 to 8, at 30 to 40°C, for 3 to 12 days.

Ubiquinone may be added to the medium initially or during the culture.

If necessary, antibiotics such as streptomycin and penicillinmaybe added to the medium, during culture. Further, control of dissolved oxygen concentration, pH control, temperature control, agitation and the like may be carried out according to general methods for use in animal cell culture.

2. Repeated batch culture

Repeated batch culture is usually carried out in a medium for use in animal cell culture at pH 6 to 8, at 30 to 40°C, for 2 to 5 days per one batch culture. Ubiquinone may be added to the medium initially or during the culture.

If necessary, antibiotics such as streptomycin and penicillin may be added to the medium, during the culture. Further, control of dissolved oxygen concentration, pH control, temperature control, agitation and the like may be carried out according to general methods for use in animal cell culture.

Upon completion of the batch culture, a given amount of a fresh medium is added to a part of the liquid culture, and the batch culture is repeated a given number of times. Then, the culture is completed. Herein, the cell grown in the batch culture is used for a seed cell for the following batch culture.

3. Fed-batch culture

Fed-batch culture is usually carried out in a medium for use in animal cell culture at pH 6 to 8, at 30 to 40°C, for 3 to 20 days.

Ubiquinone may be added to the medium initially or during the culture.

If necessary, antibiotics such as streptomycin and penicillin may be added to the medium during the culture. Further, control of dissolved oxygen concentration, pH control, temperature control, agitation and the like may be carried out according to general methods for use in animal cell culture.

In case of adding physiologically active substances, nutrient factors and the like for fed-batch culture, they are preferably added at higher concentrations than the concentrations for their routine use. For example, the physiologically active substances, the nutrient factors and the like are added in one portion at a volume 1/30- to 1/3-fold, preferably 1/20- to 1/5-fold volume of the medium. In case of addition to the medium during the culture, they are preferably added continuously or in several to several tens portions. The fed-batch culture comprising adding the physiologically active substances, the nutrient factors and the like continuously or intermittently in small quantities can attain high cellular metabolic rate and can prevent the reduction of the finally attained cell density of the culture cells due to the accumulation of metabolic waste in the culture. Because the concentration of the desired substance recovered in the fed-batch culture is higher than that in the batch culture, the useful substance can be easily separated and purified in the fed-batch culture. Thus, the productivity of the useful substance per medium is improved, compared with the batch culture.

4. Perfusion culture

Perfusion culture is usually carried out in a medium for use in animal cell culture at pH 6 to 8, at 30 to 40°C, for 10 to 40 days.

Ubiquinone may be added to the medium initially or during the culture.

If necessary, antibiotics such as streptomycin and penicillin may be added to the medium, during the culture. Further, control of dissolved oxygen concentration, pH control, temperature control, agitation and the like may be carried out according to general methods for use in animal cell culture.

As described above, a desired substance can be prepared by culturing an animal cell having an ability to produce the substance in amediumcontaining a ubiquinone until the substance is produced and accumulated in the culture, and recovering the substance therefrom.

The substances to be produced by the process of the present invention may be any substances which can be produced by animal cells and preferably substances which can be produced by cells of animals belonging to mammals.

The substances which can be produced by animal cells include substances, at least a part of which is produced by animal cells, for example, artificially modified proteins such as fusion protein or a partial fragment thereof.

The substances produced by the process of the present invention may be any substances, including immunologically functioning molecules such as an antibody, biocatalyst molecules such as enzyme, structure-forming molecules and structure-maintaining molecules such as structural protein, and vaccines against virus or the like. Suitable examples of the substances are immunologically functioning substances.

As the immunologically functioning molecules, any substances involved in the immune reaction of biological organisms such as proteins and peptides including antibodies; interferon molecules including interleukin-2 (IL-2) [Science, 193, 1007 (1976)] and interleukin-12 (IL-12) [J. Leukoc. Biol., 55, 280 (1994)]; colony-stimulating factors including granulocyte colony stimulating factor (G-CSF) [J. Biol. Chem., 258, 9017 (1983)], macrophage colony stimulating factor (M-CSF) [J. Exp. Med., 173, 269 (1992)], and granulocyte-macrophage colony stimulating factor (GM-CSF) [J. Biol. Chem., 252, 1998 (1977)]; and growth factors including erythropoietin (EPO) [J. Biol. Chem., 252, 5558 (1977)] and thrombopoietin (TPO) [Nature, 369, 533 (1994)] are mentioned.

The antibodies include antibodies secreted from hybridomas prepared by fusing a cell of an immunized animal and a myeloma cell, and antibodies prepared by genetic engineering techniques, namely antibodies prepared from cells obtained by transfecting an antibody-expression vector carrying the antibody encoding genes into host cells.

The antibodies include antibodies produced by hybridomas, humanized antibodies, human antibodies, and single chain antibodies.

The humanized antibodies include chimeric humanized antibody and complementarity determining region (hereinafter referred to as CDR)-grafted humanized antibody.

The chimeric humanized antibody means an antibody comprising the heavy-chain variable region (the heavy chain and the variable region are referred to as H-chain and as V region, respectively hereinafter, and thus this heavy-chain variable region is also referred to as VH) of a non-human animal antibody, and the light-chain variable region (the light chain is referred to as L-chain, hereinafter, and thus the region is also referred to as VL) of the non-human animal antibody, and the heavy-chain constant region (the constant region is referred to as C region, hereinafter, and thus the region is also referred to as CH) of a human antibody and the light-chain constant region (hereinafter referred to as CL) of the human antibody.

As the non-human animals, any animals from which hybridomas can be prepared, such as mouse, rat, hamster and rabbit, can be used.

The chimeric humanized antibody can be prepared by isolating the cDNAs encoding the VH and VL from a hybridoma producing monoclonal antibody, individually inserting the cDNAs into an expression vector carrying the genes encoding the human antibody CH and human antibody CL for a host cell to construct a chimeric humanized antibody expression vector, transfecting said expression vector into the host cell and culturing the resulting transformant.

As the CH for the chimeric humanized antibodies, any CH of antibodies belonging to human immunoglobulin (hereinafter referred to as human Ig), preferably the CH of antibodies belonging to human IgG class may be used. The CH of the antibodies belonging to human IgG subclass such as human IgG1, human IgG2, human IgG3 and human IgG4 can be preferably used. As the CL of the chimeric humanized antibodies, any CL of antibodies belonging to human Ig, for example, κ class or λ class, may be used.

The CDR-grafted humanized antibody means an antibody prepared by grafting the amino acid sequences of the CDRs in the VH and VL of a non-human animal antibody into appropriate sites in the VH and VL, respectively, of a human antibody.

The CDR-grafted humanized antibody can be prepared by constructing cDNAs encoding V regions in which the CDR sequences of the VH and VL of a non-human animal antibody are grafted into the CDR regions of the VH and VL of an human antibody, individually inserting the resulting cDNAs into an expression vector carrying the genes encoding human antibody CH and human antibody CL for a host cell to construct a CDR-grafted humanized antibody expression vector, and then transfecting the expression vector into the host cell to express the CDR-grafted humanized antibody. The CDR-grafted humanized antibody can be produced by culturing the resulting transformant obtained by transfecting the expression vector into the host cell.

As the CH for the CDR-grafted humanized antibodies, any CH of antibodies belonging to human Ig, preferably the CH of antibodies belonging to human IgG class may be used. The CH of the antibodies belonging to human IgG subclass such as human IgG1, human IgG2, human IgG3 and human IgG4 can be preferably used. As the CL of the CDR-grafted humanized antibodies, any CL of antibodies belonging to human Ig, for example, κ class or λ class, may be used.

The single chain antibody can be prepared by the following manner. A single chain antibody expression vector is constructed by linking a DNA encoding VH and a DNA encoding VL together with a linker, and inserting the resultant product into an expression vector for a host cell. The single chain antibody can be produced by culturing the transformant obtained by transfecting the expression vector into the host cell.

Specific examples of the antibodies include an antibody against ganglioside GD3 (hereinafter referred to as an anti-GD3 antibody), an antibody against chemokine receptor CCR4 (hereinafter referred to as an anti-CCR antibody), an antibody against human interleukin-5 receptor α-chain (hereinafter referred to as an anti-IL-5 receptor α-chain antibody) and an antibody against ganglioside GM2 (hereinafter referred to as an anti-GM2 antibody). The anti-GD3 antibodies include chimeric humanized anti- GD3 antibody (hereinafter referred to as chimeric anti-GD3antibody) KM-871 (Japanese Published Unexamined Patent Application No. 304989/93) and CDR-grafted humanized anti-GD3 antibody KM8871 (WO 01/23432). The anti-CCR4 antibodies include anti-CCR4 chimeric humanized antibody (hereinafter referred to as chimeric anti-CCR4 antibody) KM2760 (WO 01/65754). The anti-IL-5 receptor α-chain antibodies include chimeric humanized anti-IL-5 receptor α-chain antibodies KM1399 and KM7399 (WO 97/10354) and CDR-grafted humanized anti-IL-5 receptor α-chain antibodies KM8399 and KM9399 (WO 97/10354). The anti-GM2 antibodies include CDR-grafted humanized anti-GM2 antibodies KM8966, KM 8967, KM8969 and KM8970 (Japanese Published Unexamined Patent Application No. 257893/98).

The biocatalyst molecules include enzymes and ribozymes.

The structure-forming molecules or structure-maintaining molecules include keratin, collagen, elastin, resilin and fibroin.

The vaccines include smallpox vaccine, polio vaccine, measles vaccine, rubella vaccine, mumps vaccine, rabies vaccine, and varicella vaccine. The virus vaccines for animals include bovine ephemeral fever vaccine, Ibaraki disease vaccine and bovine infectious bronchitis vaccine.

On the completion of culturing, the desired substance can actively be secreted outside the host cell, according to themethodof Paulson, et al. [J. Biol. Chem., 264, 17619 (1989)], the method of Lowe, et al. [Proc. Natl. Acad. Sci. USA, 86, 8227 (1989); Genes Develop., 4, 1288 (1990)] or the methods described in Japanese Published Unexamined Patent Application No. 336963/97, WO 94/23021 and the like. Namely, the desired substance can be secreted outside the host cell by expressing the substance with a signal peptide added to its N-terminal using genetic engineering techniques.

The substances produced by the process of the present invention can be isolated and purified by general isolation and purification methods of substances such as proteins.

When the substance produced by the method of the present invention is expressed in a soluble form intracellularly, the cell is recovered by centrifugation after the completion of culturing, which is suspended in an aqueous buffer, and is then disrupted with sonicator, French Press, Manton-Gaulin homogenizer, Dynomill and the like, to recover a cell-free extract. From the supernatant obtained by centrifuging the cell-free extract, a partially purified product or a purified product can be recovered, by using general methods for isolation and purification of substances, namely solvent extraction, salting-out with ammonium sulfate and the like, desalting, precipitation with organic solvents, anion exchange chromatography using resins such as diethylaminoethyl (DEAE)-Sepharose, DIAION HPA-75 (manufactured by Mitsubishi Chemical Corporation), cation exchange chromatography using resins such as S-Sepharose FF (manufactured by Amersham Biosciences), hydrophobic chromatography using resins such as butyl Sepharose and phenyl Sepharose, gel filtration using molecular sieve, affinity chromatography using protein A, and electrophoresis such as isoelectric focusing, singly or in combination.

When the substance produced by the method of the present invention is secreted extracellularly, the useful substance can be recovered in the culture supernatant. In other words, the culture is treated by the same methods as described above, such as centrifugation, to recover the culture supernatant. From the culture supernatant, a partially purified product or a purified product can be recovered, by use of the same isolation and purification methods as described above.

In accordance with the present invention, the productivity of the substance can be improved by increasing the specific production rate (SPR) of the substance.

The addition of ubiquinone to the medium as described above can improve the substance productivity per cell of an animal cell through the increase of the stress tolerance of the animal cell against the culture environment, and the activation of the biosynthesis or secretion of substances such as protein.

Examples of the present invention are described below.

Best Modes for Carrying Out the Invention

Example 1

Production of an antibody using a Q10-containing medium (1)

The following batch culture was performed with transformant cell line 61-33γ (FERM BP-7325, WO 01/29246) which was obtained by using hybridoma cell line YB2/0 derived from a rat myeloma cell line as a host cell and produces a chimeric anti-GD3 antibody.

A health drink SANOMIT (manufactured by MSE Pharmazeutica GmbH) comprising Q10, water, glycerin, ethanol and lecithin was added to a serum-free medium [Hybridoma-SFM (manufactured by Invitrogen Corporation) containing 0.2 %(w/v) bovine serum albumin, 20 mg/L insulin, 20 mg/L transferrin and 200 nmol/L methotrexate (hereinafter abbreviated as MTX)] to a final Q10 concentration of 500 μM, and 30 mL of the resulting serum-free medium was placed in a T-225 $cm^2$ flask. The cells were inoculated in the flask to a cell density of $3 \times 10^5$ cells/mL, followed by stationary culture in a 5 %-$CO_2$ incubator at 37°C for 6 days (Q10-added culture). Additionally, the cells were also subjected to stationary culture for 6 days by the same manner as described above, except for no addition of the SANOMIT (manufactured by MSE Pharmazeutica GmbH) to the serum-free medium (Q10-free culture).

From the start to the completion of culturing, the liquid culture was sampled once a day, to assay the viable cell density (cells/mL) and the antibody concentration (μg/mL) respectively. Further, the viable cell density was measured by a dye-exclusion method using 0.4% trypan blue solution.

The cumulative number of viable cells is shown as the total sum of the viable cell density multiplied by the time as elapsed. In the present Example, the viable cell density was measured once a day, and the cumulative number of viable cells was shown as value of the additive sum of the individual viable cell densities measured each time (cells/mL x days).

Additionally, the specific antibody production rate and the relative specific antibody production rate were calculated respectively by the following equations.

Specific antibody production rate (μg/cell/day) =

antibody concentration (μg/mL) ÷ the cumulative number of

viable cells (cells/mL $\times$ days)

Relative specific antibody production rate (%) =

(specific antibody production rate in Q10-added culture) ÷

(specific antibody production rate in Q10-free culture) $\times$ 100.

Consequently, the cumulative number of cells on the completion of culturing was determined to be $1.71 \times 10^7$ cells/mL $\times$ days in the Q10-free culture and to be $1.51 \times 10^7$ cells/mL $\times$ days in the Q10-added culture, respectively. Thus, no significant difference in cumulative number of cells was observed, even when Q10 was added to the medium.

In contrast, the relative specific antibody production rates in the Q10-free culture and in the Q10-added culture were determined to be 100 % and 166.3 %, respectively, on the completion of culturing. Thus, the specific antibody production rate was increased by adding Q10 to the medium.

Example 2

Production of an antibody using a Q10-containing medium (2)

The following fed-batch culture was performed with transformant cell line 61-33$\gamma$ (FERM BP-7325) which was obtained by using hybridoma cell line YB2/0 derived from a rat myeloma cell line as a host cell and produces a chimeric anti-GD3 antibody.

In a T-225 $cm^2$ flask charged with 30 mL of a serum-free medium (Hybridoma-SFM; manufactured by Invitrogen Corporation), the cells were inoculated to a cell density of $3 \times 10^5$ cells/mL, followed by stationary culture in a 5 %-$CO_2$ incubator at $37°C$ for 3 days.

On the completion of culturing, the resulting cells were inoculated in a 1-liter spinner flask (manufactured by Shibata Hario, Co., Ltd.) containing 0.7 liter of Hybridoma-SFM to $3 \times 10^5$ cells/ml. While adjusting the liquid culture to pH $7.1 \pm 0.1$ and the dissolved oxygen concentration to $3 \pm 0.2$ ppm, culturing was carried out at $37°C$ for 11 days under the condition of stirring at 50 rpm. Aeration was carried out via a porous polytetrafluoroethylene tube arranged in the spinner flask, and a mixture gas of air, oxygen and carbon dioxide was supplied. Additionally, the pH was controlled by changing the ratio of air and carbon dioxide and by feeding 1 mol/L sodium carbonate solution. Furthermore, the dissolved oxygen concentration was controlled by changing the ratio of air and oxygen.

For the Q10-added culture, a solution of Q10 dissolved in Tween 80 was added to the Hybridoma-SFM to a Q10 concentration of 50 $\mu$mol/L, while for the Q10-free culture, Tween 80 of the same volume was added to the Hybridoma-SFM.

For the purpose of compensating the consumed amino acids estimated on the basis of the specific consumption rate, a feed medium comprising amino acids (0.140 g/L L-alanine, 0.470 g/L L-arginine monohydrochloride, 0.159 g/L L-asparagine monohydrate, 0.168 g/L L-aspartic acid, 0.511 g/L L-cystine dihydrochloride, 0.420 g/L L-glutamic acid, 4.677 g/L L-glutamine, 0.168 g/L glycine, 0.235 g/L L-histidine monohydrochloride dihydrate, 0.588 g/L L-isoleucine, 0.588 g/L L-leucine, 0.818 g/L L-lysine monohydrochloride, 0.168 g/L L-methionine, 0.370 g/L L-phenylalanine, 0.224 g/L L-proline, 0.235 g/L L-serine, 0.532 g/L L-threonine, 0.090 g/L L-tryptophan, 0.581 g/L L-tyrosine disodium dihydrate, and 0.526 g/L L-valine), vitamins (0.0728 mg/L d-biotin, 0.0224 g/LcalciumD-pantothenate, 0.0224 g/L choline chloride, 0.0224 g/Lfolicacid, 0. 0403 g/Lmyo-inositol, 0. 0224 g/Lniacin amide, 0.0224 g/L pyridoxal hydrochloride, 0.00224 g/L riboflavin, 0.0224 g/L thiamine hydrochloride, and 0.0728 mg/L cyanocobalamine), 0.2 g/L insulin, 0.2 g/L transferrin, and 1.6 g/L bovine serum albumin, which were adjusted to higher concentrations than routine concentrations for addition, was added in 0.07-L portions once a day or at less frequency when the viable cell density was more than $4 \times 10^6$ cells/ml $\times$ days. In other words, 0.07 L of the feed medium was added on 3rd, 5th, 6th, 7th and 8th days after the start of culturing. On 3rd day after the start of culturing and thereafter, 100 g/L glucose solution was added in appropriate timing so that the glucose concentration in the liquid culture immediately after addition might be 2,500 mg/L.

From the start to the completion of culturing, the liquid culture was sampled once a day to individually measure the viable cell density (cells/mL) and antibody concentration ($\mu$g/mL), to calculate the cumulative number of viable cells, the specific antibody production rate and the relative specific antibody production rate according to the method described in Example 1.

Consequently, the cumulative number of cells on the completion of culturing was $2.97 \times 10^7$ cells/ml $\times$ days in the Q10-free culture and $3.98 \times 10^7$ cells/ml $\times$ days in the Q10-added culture, respectively. Thus, no significant change was observed in cumulative number of cells even when Q10 was added to the medium.

In contrast, the relative specific antibody production rate on the completion of the culturing was 100 % in the Q10-free culture and 137.1 % in the Q10-added culture, respectively. Thus, the specific antibody production rate was increased by adding Q10 to the medium.

Example 3

Production of an antibody using a Q10-containing medium (3)

Lens culinaris agglutinin (hereinafter abbreviated as LCA) resistant cell line CHO-LCA, which is derived from Chinese hamster ovary cell line CHO/DG44, was prepared by the method described in WO 02/31140, and chimeric anti-CCR4 antibody expression vector pCANTEX 2160 (WO 01/64754) was transfected into this cell line. Then, a methotrexate (MTX)-resistant cell was selected for gene amplification to obtain transformant cell line 503LCA500D (FERM BP-8239) producing chimeric anti-CCR4 antibody. The following fed-batch culture was performed using this cell line 503LCA500D. 503LCA500D was deposited as FERM BP-8239 in International Patent Organism Depositary, National Institute of Advanced Industrial Science and Technology (AIST Tsukuba Central 6, 1-1-1 Higashi, Tsukuba, Ibaraki, Japan) as of November 20, 2002.

The cells were inoculated in a 250-mL conical flask containing 50 mL of a serum-free medium (EX-CELL 302 medium, manufactured by JRH Biosciences) to a cell density of $3 \times 10^5$ cells/mL, followed by spinner culture at 100 rpm at 37°C.

In Q10-added cultures, 0.5 mL or 1 mL of a health drink SANOMIT (manufactured by MSE Pharmazeutica GmbH) comprising Q10, water, glycerin, ethanol and lecithin was added to 20 mL of an animal-protein-material-free feed medium comprising amino acids (0.22 g/L L-alanine, 0.74 g/L L-arginine monohydrochloride, 0.22 g/L L-asparagine (anhydrous), 0.26 g/L L-aspartic acid, 0.8 g/L L-cystine dihydrochloride, 0.66 g/L L-glutamic acid, 7.34 g/L L-glutamine, 0.26 g/L glycine, 0.37 g/L L-histidine monohydrochloride dihydrate, 0.92 g/L L-isoleucine, 0.92 g/L L-leucine, 1.29 g/L L-lysine monohydrochloride, 0.26 g/L L-methionine, 0.58 g/L L-phenylalanine, 0.35 g/L L-proline, 0.37 g/L L-serine, 0.84 g/L L-threonine, 0.14 g/L L-tryptophan, 0.92 g/L L-tyrosine disodium dihydrate, and 0.83 g/L L-valine), vitamins (0.11 mg/L d-biotin, 0.035 g/L calcium D-pantothenate, 0.035 g/L choline chloride, 0.035 g/L folic acid, 0.063 g/L myo-inositol, 0.035 g/L niacin amide, 0.035 g/L pyridoxal hydrochloride, 0.0035 g/L riboflavin, 0.035 g/L thiamine hydrochloride, and 0. 00011 mg/L cyanocobalamine), 0.0251 g/L ethanolamine, 0.314 g/L insulin, 8 g/L soybean hydro lys ate HY-SOY (manufactured by Quest International), 0.05 g/L ethylenediaminetetraacetic acid ferric sodium salt (manufactured by Sigma-Aldrich Co.), and 2 mL cholesterol lipid concentrate (1,000 $\times$ aqueous solution, manufactured by Invitrogen Corp.). The concentrations of Q10 in the feed media after addition of SANOMIT were 1,563 μmol/L and 3,125 μmol/L respectively. To the cultures were added the 0.5 mL-SANOMIT-added feed medium in the Q10-added culture 1, the 1 mL-SANOMIT-added feed medium in the Q10-added culture 2, and the SANOMIT-free feed medium in the Q10-free culture respectively in 4.2 mL portions on 3rd, 5th, 7th and 9th days after the start of culturing. By the addition of the feed media, the concentration of Q10 in the medium became from 121 to 393 μmol/L in the Q10-added culture 1 and from 242 to 786 μmol/L in the Q10-added culture 2. On 3rd day after the start of culturing and thereafter, 200 g/L glucose solution was added in appropriate timing so that the glucose concentration in the culture immediately after addition might be 4,000 mg/L. Culturing was performed for 13 days in the Q10-added culture 1 and the Q10-free culture, and for 15 days in the Q10-added culture 2.

From 3rd day after the start of culturing to the completion of culturing, the culture was sampled every three days to individually measure the viable cell density (cells/mL) and the antibody concentration (μg/mL) and calculate the cumulative number of viable cells, the specific antibody production rate and the relative specific antibody production rate according to the method described in Example 1.

Consequently, the cumulative number of cells on the completion of culturing was $3.94 \times 10^7$ cells/mL $\times$ days in the Q10-free culture, $3.64 \times 10^7$ cells/mL $\times$ days in the Q10-added culture 1 and $2.00 \times 10^7$ cells/mL $\times$ days in the Q10-added culture 2. Thus, no significant change was observed in cumulative number of cells even when Q10 was added to the medium.

In contrast, the relative specific antibody production rate on the completion of culturing was 100 % in the Q10-free culture, 128.8% in the Q10-added culture 1 and 149.1 % in the Q10-added culture 2 which has the high concentration of Q10 added . Thus, the specific antibody production rate was increased dependently on the concentration of added Q10.

Example 4

Production of an antibody using a Q10-containing medium (4)

The batch culture was performed using transformant cell line NSO/LCA-CCR4 (FERM BP-7964) producing chimeric anti-CCR4 antibody as prepared in Reference Example 1.

The cells were inoculated in a serum fraction-addedmedium [RPMI 1640 medium (manuf actured by Invitrogen Corp.) containing 10% Daigo GF-21 (manufactured by NIHON PHARMACEUTICAL CO. , LTD.) and 500 nM MTX (manufactured by Sigma-Aldrich Corp.)] to which a health drink SANOMIT (manufactured by MSE Pharmazeutica GmbH) comprising Q10, water, glycerin, ethanol and lecithin had been added to a Q10 final concentration of 100 μmol/L(Q10-added culture) and in a serum fraction-added medium free of Q10 (Q10-free culture) to a cell density of $3 \times 10^5$ cells/mL, followed by stationary culture in a 5 %-$CO_2$ incubator at 37°C.

On 4th day from the start of culturing, the culture was sampled to assay the viable cell density (cells/mL) and the antibody concentration (μg/mL) respectively.

The cumulative number of viable cells is shown as the total sum of the viable cell density multiplied by the time as elapsed. In the present Example, as the viable cell density was measured once, the cumulative number of viable cells was calculated by the following equation as a trapezoidal area formed by the change of the cell density with time related to the inoculated cell density and the measured cell density.

$$\text{Cumulative number of viable cells (cells/mL} \times \text{days)} =$$

$$(\text{inoculated cell density + measured cell density}) \times$$

$$\text{time as elapsed} \div 2$$

The specific antibody production rate and the relative specific antibody production rate were calculated respectively by the method described in Example 1.

Consequently, the cumulative number of cells on the completion of culturing was determined to be $1.59 \times 10^6$ cells/mL $\times$ days in the Q10-free culture and to be $1.34 \times 10^6$ cells/mL $\times$ days in the Q10-added culture, respectively. Thus, no significant difference in cumulative number of cells was observed even when Q10 was added to the medium.

In contrast, the relative specific antibody production rate on the completion of culturing was 100 % in the Q10-free culture and 131.9 % in the Q10-added culture, respectively. Thus, the specific antibody production rate was increased by adding Q10 to the medium.

Reference Example 1

Preparation of chimeric anti-CCR4 antibody producing transformant cell line NSO/LCA-CCR4

(1) Preparation of LCA-resistant cell line of NSO

Mouse myeloma cell line NSO (Riken Cell Bank: RCB 0213) was cultured in RPMI 1640 medium (manufactured by Invitrogen Corp.) containing a fetal bovine serum (manufactured by Invitrogen Corp.) at a volume ratio of 10 % (hereinafter referred to as RPMI-FBS (10)) in a flask (75 cm$^2$) for suspension culture (manufactured by Iwaki Glass Co., Ltd.), and was proliferated till just before being confluent. RPMI-FBS (10) medium was added to the cells in the culture to a density of $1 \times 10^5$ cells/ml for suspension. Then, 0.1 µg/ml of N-methyl-N'-nitro-N-nitrosoguanidine (hereinafter referred to as MNNG, manufactured by Sigma-Aldrich Co. ) as an alkylating agent was added or not added. The cells were allowed to stand in a $CO_2$ incubator (manufactured by TABAI) at 37°C for 3 days, and then inoculated in a 96-well plate for suspension culture (manufactured by Iwaki Glass Co., Ltd.) to a density of 1,000 cells/well. LCA (manufactured by Vector) at a final concentration of 1 mg/ml in the medium was added to each well. After the culturing in a $CO_2$ incubator at 37°C for 2 weeks, LCA-resistant colonies that appeared were obtained as an LCA-resistant cell line of NSO which was designated NS0-LCA.

(2) Preparation of cells expressing a chimeric anti-CCR4 antibody

Cells producing a chimeric anti-CCR4 antibody stably were prepared in the following manner using pKANTEX2160, which is a tandem expression vector for chimeric anti-CCR4 antibody, described in WO 01/64754.

$4 \times 10^6$ cells of NS0-LCA was transfected with 10 µg of chimeric anti-CCR4 antibody expression vector pKANTEX2160 by electroporation [Cytotechnology, 3, 133, (1990)], and suspended in 10 ml of RPMI-FBS (10) medium. The suspension was dispensed into a 96-well culture plate (manufactured by Sumitomo Bakelite Co., Ltd.) at 200 µl/ well. After culturing was performed in a 5 % $CO_2$ incubator at 37°C for 24 hours, G418 was added in an amount of 0.5 mg/ml. The culturing was performed for from 1 to 2 weeks. Colonies of a transformant showing G418 resistance appeared. The culture supernatant was recovered from the wells with the proliferation observed, and the amount of chimeric anti-CCR4 antibody accumulated in the culture supernatant was measured by ELISA described in Reference Example 1(3).

With respect to the transformant in the well where the production of chimeric anti-CCR4 antibody was observed in the culture supernatant, the cells were suspended in RPMI-FBS (10) medium containing 0.5 mg/ml of G418 and 50 nmol/L of MTX to a cell density of 1 to $2 \times 10^5$ cells/ml and 2 ml of the suspension was dispensed into each well of 24-well plate (manufactured by Greiner GmbH) to increase the amount of the antibody by using a dihydrofolate reductase gene amplification system. The culturing was performed in a 5 % $CO_2$ incubator at 37°C for from 1 to 2 weeks to induce a transformant showing 50 nmol/L MTX resistance. The expression level of chimeric anti-CCR4 antibody in the culture supernatant in the well where the proliferation of the transformant was observed was measured by ELISA described in Reference Example 1(3). With respect to the transformant in the well where the production of chimeric anti-CCR4 antibody was observed in the culture supernatant, the MTX concentration was increased to 200 nmol/L and 500 nmol/L according to the above method, and one transformant capable of proliferation in RPMI-FBS (10) medium finally containing 0.5 mg/ml of G418 and 500 nmol/L of MTX and producing chimeric anti-CCR4 antibody at a high level was selected, and designated NS0/LCA-CCR4. Incidentally, NSO/LCA-CCR4 was deposited as FERM BP-7964 in International Patent Organism Depositary, National Institute of Advanced Industrial Science and Technology (AIST Tsukuba Central 6, 1-1-1 Higashi, Tsukuba, Ibaraki, Japan) as of March 14, 2002.

(3) Quantitative determination of an antibody in a culture supernatant by IgG-ELISA

The concentration of chimeric anti-CCR4 antibody in the culture supernatant was measured as follows.

1 mg of anti-human IgG antibody (manufactured by American Qualex) was dissolved in 1,200 ml of PBS. 50 $\mu$l of the solution was charged in each well of a 96-well ELISA plate (manufactured by Greiner GmbH), and allowed to stand overnight at 4°C. Then, the solution in each well was discarded, and PBS containing 1 % bovine serum albumin (manufactured by Sigma-Aldrich Co.)(hereinafter referred to as 1 % BSA-PBS) was added in an amount of 100 $\mu$l/well. The reaction was performed at room temperature for 1 hour to block the remaining active groups. 1 % BSA-PBS was discarded, and various dilute solutions of the culture supernatant of the transformant were added in amounts of 50 $\mu$l/ well. The reaction was performed at room temperature for 1 hour. After the reaction, each well was washed with PBS containing 0.05 % Tween 20 (manufactured by Wako Pure Chemical Industries, Ltd.)(hereinafter referred to as Tween-PBS). A peroxidase-labeled goat anti-human IgG (H & L) antibody solution (manufactured by American Qualex) diluted to 3,000 times with 1 % BSA-PBS was then added in an amount of 50 $\mu$l/well as a secondary antibody solution, and the reaction was performed at room temperature for 1 hour. After the reaction, each well was washed with Tween-PBS, and an ABTS substrate solution [solution prepared by dissolving 0.55 g of ammonium 2,2'-azino-bis (3-ethylbenzothiazoline-6-sulfonate) in 1 L of 0.1 mol/L citrate buffer (pH 4.2) and adding a 30 % hydrogen peroxide solution in an amount of 1 $\mu$l/mL just before use] was added in an amount of 50 $\mu$l/well for color development, and an absorbance at 415 nm was measured with a microplate reader (manufactured by Bio-Rad).

Industrial Applicability

The present invention provides a process for producing a substance efficiently by use of an animal cell.

| Applicant's or agent's file reference 1436 | International application No. |
|---|---|

# INDICATIONS RELATING TO DEPOSITED MICROORGANISM OR OTHER BIOLOGICAL MATERIAL

## (PCT Rule 13bis)

**A.** The indications made below relate to the deposited microorganism or other biological material referred to in the description on page 18 , line 7 .

**B. IDENTIFICATION OF DEPOSIT**        Further deposits are identified on an additional sheet ☐

Name of depositary institution

International Patent Organism Depositary, National Institute of Advanced Industrial Science and Technology

Address of depositary institution *(including postal code and country)*

AIST Tsukuba Central 6, 1-1, Higashi 1-Chome Tsukuba-shi, Ibaraki-ken 305-8566 Japan

| Date of deposit 20.11.02 | Accession Number FERM BP-8239 |
|---|---|

**C. ADDITIONAL INDICATIONS** *(leave blank if not applicable)*     This information is continued on an additional sheet ☐

Until the publication of the mentioned of the grant of the European Patent, or until the date on which the application has been refused or withdrawn or is deemed to be withdrawn, the furnishment of a deposited microorganism shall be effected only by the issue of a sample of the deposited microorganism to an expert nominated by the requester, in each of the designated states where protection by the European patent is sought. (Rule 28(4))

**D. DESIGNATED STATES FOR WHICH INDICATIONS ARE MADE** *(if the indications are not for all designated States)*

**E. SEPARATE FURNISHING OF INDICATIONS** *(leave blank if not applicable)*

The indications listed below will be submitted to the International Bureau later *(specify the general nature of the indications e.g., "Accession Number of Deposit")*

| ━━━ For receiving Office use only ━━━ | ━━━ For International Bureau use only ━━━ |
|---|---|
| ☐ This sheet was received with the international application | ☐ This sheet was received by the International Bureau on: |
| Authorized officer | Authorized officer |

Form PCT/RO/134 (July1998; reprint January 2004)

**Claims**

1. A process for producing a substance, which comprises culturing an animal cell having an ability to produce the substance in a medium containing a ubiquinone thereby to produce and accumulate the substance in the culture, and recovering the substance therefrom.

2. The process according to claim 1, wherein the ubiquinone is Q10.

3. The process according to claim 1 or 2, wherein the animal cell is a cell of an animal belonging to mammals.

4. The process according to any one of claims 1 to 3, wherein the animal cell is a myeloma cell, a cell derived from the myeloma cell or an ovarian cell.

5. The process according to any one of claims 1 to 4, wherein the cell is a cell transformed with a recombinant vector carrying a gene involved in the production of the substance.

6. The process according to any one of claims 1 to 5, wherein the medium is a serum-free medium or a protein-free medium.

7. The process according to any one of claims 1 to 6, wherein the culturing is carried out by batch culture, repeated batch culture, fed-batch culture or perfusion culture.

8. The process according to any one of claims 1 to 7, wherein the substance is selected from an immunologically functional molecule, a biocatalyst molecule, a structure-forming molecule, a structure-maintaining molecule or a vaccine.

9. The process according to claim 8, wherein the immunologically functional molecule is an antibody.

10. A method of improving the productivity of a substance, which comprises culturing an animal cell having an ability to produce the substance in a medium containing a ubiquinone.

11. The method according to claim 10, wherein the ubiquinone is Q10.

12. The method according to claim 10 or 11, wherein the animal cell is a cell of an animal belonging to mammals.

13. The method according to any one of claims 10 to 12, wherein the animal cell is a myeloma cell, a cell derived from the myeloma cell or an ovarian cell.

14. The method according to any one of claims 10 to 13, wherein the cell is a cell transformed with a recombinant vector carrying a gene involved in the production of the substance.

15. The method according to any one of claims 10 to 14, wherein the medium is a serum-free medium or a protein-free medium.

16. The method according to any one of claims 10 to 15, wherein the culturing is carried out by batch culture, repeated batch culture, fed-batch culture or perfusion culture.

17. The method according to any one of claims 10 to 16, wherein the substance is selected from an immunologically functional molecule, a biocatalyst molecule, a structure-forming molecule, a structure-maintaining molecule or a vaccine.

18. The method according to claim 17, wherein the immunologically functional molecule is an antibody.

<div align="center">**INTERNATIONAL SEARCH REPORT**</div>

| | International application No. |
|---|---|
| | PCT/JP02/12522 |

**A. CLASSIFICATION OF SUBJECT MATTER**
Int.Cl⁷ C12N15/09, C12N5/10, C12P21/02

According to International Patent Classification (IPC) or to both national classification and IPC

**B. FIELDS SEARCHED**

Minimum documentation searched (classification system followed by classification symbols)
Int.Cl⁷ C12N15/09, C12N5/10, C12P21/02

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)
MEDLINE(STN), WPI(DIALOG), BIOSIS(DIALOG), JICST FILE(JOIS)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | EP 60565 A (MAX PLANCK GES FOERDERUNG WISSENSCHAFTEN),<br>22 September, 1982 (22.09.82),<br>Full text<br>& DE 3110559 A  & JP 57-170186 A | 1-3,6<br>4-5,7-9<br>10-18 |
| X<br>Y<br>A | EP 120722 A (PARFUMS DIOR SACHRISTIAN),<br>10 August, 1984 (10.08.84),<br>Full text<br>& FR 2540381 A  & JP 59-152333 A | 1-3,6<br>4-5,7-9<br>10-18 |
| Y<br>A | Osamu KANEMITSU, "Kotai Kogaku Nyumon",<br>Chijinshokan Co., Ltd., 25 January, 1994 (25.01.<br>94), pages 202 to 204 | 4,5,8,9<br>13,14,17,18 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 February, 2003 (10.02.03) | 25 February, 2003 (25.02.03) |

| Name and mailing address of the ISA/<br>Japanese Patent Office | Authorized officer |
|---|---|
| Facsimile No. | Telephone No. |

Form PCT/ISA/210 (second sheet) (July 1998)

**INTERNATIONAL SEARCH REPORT**

| International application No. |
| --- |
| PCT/JP02/12522 |

**C (Continuation).   DOCUMENTS CONSIDERED TO BE RELEVANT**

| Category* | Citation of document, with indication, where appropriate, of the relevant passages | Relevant to claim No. |
| --- | --- | --- |
| Y<br>A | Edited by The Japanese Biochemical Society, "Shin Seikagaku Jikken Koza (Vol.18) Saibo Baiyo Gijutsu", Tokyo Kagaku Dojin, 20 October, 1990 (20.10.90), pages 303 to 323 | 7<br>16 |

Form PCT/ISA/210 (continuation of second sheet) (July 1998)